# EUROPÄISCHE PATENTSCHRIFT

(11) **EP 1 629 116 B1**
(45) Veröffentlichungstag und Bekanntmachung des Hinweises auf die Patenterteilung: **10.09.2008**
(21) Anmeldenummer: 04739451.5
(22) Anmeldetag: 28.05.2004
(51) Int. Cl.: C12Q 1/68, B01L 3/00

(54) **VERFAHREN ZUR DETEKTION VON DNA-PUNKTMUTATIONEN (SNP-ANALYSE) SOWIE ZUGEHÖRIGE ANORDNUNG**
METHOD FOR DETECTING DNA POINT MUTATIONS (SINGLE NUCLEOTIDE POLYMORPHISM (SNP) ANALYSIS) AND ASSOCIATED ARRANGEMENT
PROCEDE DE DETECTION DE MUTATIONS PONCTUELLES D'ADN (ANALYSE DE PNS) ET SYSTEME ASSOCIE

(30) Priorität: 30.05.2003 DE 10324912
(43) Veröffentlichungstag der Anmeldung: 01.03.2006
(73) Patentinhaber: SIEMENS AKTIENGESELLSCHAFT, 80333 München (DE)
(72) Erfinder: GUMBRECHT, Walter, 91074 Herzogenaurach (DE); PAULICKA, Peter, 91056 Erlangen (DE); STANZEL, Manfred, 91056 Erlangen (DE)
(86) Internationale Anmeldenummer: PCT/EP2004/005829
(87) Internationale Veröffentlichungsnummer: WO 2004/106546

(56) Entgegenhaltungen:
- WO-A-02/083952
- DE-A- 10 111 420
- US-A- 5 965 410
- US-B1- 6 391 558
- MAO H ET AL: "REUSABLE PLATFORMS FOR HIGH-THROUGHPUT ON-CHIP TEMPERATURE GRADIENT ASSAYS" ANALYTICAL CHEMISTRY, AMERICAN CHEMICAL SOCIETY. COLUMBUS, US, Bd. 19, Nr. 74, 1. Oktober 2002 (2002-10-01), Seiten 5071-5075, XP001141023 ISSN: 0003-2700
- LIU W T ET AL: "Optimization of an oligonucleotide microchip for microbial identification studies: a non-equilibrium dissociation approach." ENVIRONMENTAL MICROBIOLOGY. OCT 2001, Bd. 3, Nr. 10, Oktober 2001 (2001-10), Seiten 619-629, XP002295897 ISSN: 1462-2912
- FOTIN A V ET AL: "Parallel thermodynamic analysis of duplexes on oligodeoxyribonucleotide microchips." NUCLEIC ACIDS RESEARCH. 15 MAR 1998, Bd. 26, Nr. 6, 15. März 1998 (1998-03-15), Seiten 1515-1521, XP002295898 ISSN: 0305-1048
- EL FANTROUSSI SAID ET AL: "Direct profiling of environmental microbial populations by thermal dissociation analysis of native rRNAs hybridized to oligonucleotide microarrays." APPLIED AND ENVIRONMENTAL MICROBIOLOGY. APR 2003, Bd. 69, Nr. 4, April 2003 (2003-04), Seiten 2377-2382, XP002295899 ISSN: 0099-2240
- URAKAWA HIDETOSHI ET AL: "Optimization of single-base-pair mismatch discrimination in oligonucleotide microarrays." APPLIED AND ENVIRONMENTAL MICROBIOLOGY. MAY 2003, Bd. 69, Nr. 5, Mai 2003 (2003-05), Seiten 2848-2856, XP002295900 ISSN: 0099-2240
- CHECHETKIN V R ET AL: "Sequencing by hybridization with the generic 6-mer oligonucleotide microarray: an advanced scheme for data processing." JOURNAL OF BIOMOLECULAR STRUCTURE & DYNAMICS. AUG 2000, Bd. 18, Nr. 1, August 2000 (2000-08), Seiten 83-101, XP009036300 ISSN: 0739-1102
- BREEN G: "NOVEL AND ALTERNATE SNP AND GENETIC TECHNOLOGIES" PSYCHIATRIC GENETICS, RAPID COMMUNICATIONS OF OXFORD LTD, XX, Bd. 12, Nr. 2, Juni 2002 (2002-06), Seiten 83-88, XP009001223 ISSN: 0955-8829
- KAWAGOE J L ET AL: "ENZYME-MODIFIED ORGANIC CONDUCTING SALT MICROELECTRODE" ANALYTICAL CHEMISTRY, AMERICAN CHEMICAL SOCIETY. COLUMBUS, US, Bd. 63, Nr. 24, 15. Dezember 1991 (1991-12-15), Seiten 2961-2964, XP000242061 ISSN: 0003-2700
- BERNARD ET AL: "INTEGRATED AMPLIFICATION AND DETECTION OF THE C677T POINT MUTATION IN THE METHYLENETETRAHYDROFOLATE REDUCTASE GENE BY FLUORESCENCE RESONANCE ENERGY TRANSFER AND PROBE MELTING CURVES" ANALYTICAL BIOCHEMISTRY, ACADEMIC PRESS, SAN DIEGO, CA, US, Bd. 255, Januar 1998 (1998-01), Seiten 101-107, XP002126373 ISSN: 0003-2697
- DATABASE MEDLINE [Online] US NATIONAL LIBRARY OF MEDICINE (NLM), BETHESDA, MD, US; 1996, PARIENTE F ET AL: "Enzyme support systems for biosensor applications based on gold-coated nylon meshes." XP002295906 Database accession no. NLM8828163 & BIOSENSORS & BIOELECTRONICS. 1996, Bd. 11, Nr. 11, 1996, Seiten 1115-1128, ISSN: 0956-5663
- CLOAREC J P ET AL: "Immobilization of homooligonucleotide probe layers onto Si/SiO(2) substrates: characterization by electrochemical impedance measurements and radiolabelling." BIOSENSORS & BIOELECTRONICS. MAY 2002, Bd. 17, Nr. 5, Mai 2002 (2002-05), Seiten 405-412, XP002295901 ISSN: 0956-5663
- OZKAN D ET AL: "ALLELE-SPECIFIC GENOTYPE DETECTION OF FACTOR V LEIDEN MUTATION FROM POLYMERASE CHAIN REACTION AMPLICONS BASED ON LABEL-FREE ELECTROCHEMICAL GENOSENSOR" ANALYTICAL CHEMISTRY, AMERICAN CHEMICAL SOCIETY. COLUMBUS, US, Bd. 74, Nr. 23, 1. Dezember 2002 (2002-12-01), Seiten 5931-5936, XP001161483 ISSN: 0003-2700
- NOJIMA TAKAHIKO ET AL: "Direct detection of single nucleotide polymorphism (SNP) with genomic DNA by the ferrocenylnaphthalene diimide-based electrochemical hybridization assay (FND-EHA)." ANALYTICAL SCIENCES : THE INTERNATIONAL JOURNAL OF THE JAPAN SOCIETY FOR ANALYTICAL CHEMISTRY. JAN 2003, Bd. 19, Nr. 1, Januar 2003 (2003-01), Seiten 79-83, XP009036299 ISSN: 0910-6340
- HASSMANN J ET AL: "Development of a molecular diagnosis assay based on electrohybridization at plastic electrodes and subsequent PCR" BIOSENSORS & BIOELECTRONICS, ELSEVIER SCIENCE PUBLISHERS, BARKING, GB, Bd. 16, Nr. 9-12, Dezember 2001 (2001-12), Seiten 857-863, XP002257335 ISSN: 0956-5663
- LEUNG Y F ET AL: "All aboard the chip!" TRENDS IN BIOTECHNOLOGY. NOV 2001, Bd. 19, Nr. 11, November 2001 (2001-11), Seiten 430-431, XP004309117 ISSN: 0167-7799
- SOUTEYRAND E ET AL: "Comparison between electrochemical and optoelectrochemical impedance measurements for detection of DNA hybridization." APPLIED BIOCHEMISTRY AND BIOTECHNOLOGY. 2000 NOV-DEC, Bd. 89, Nr. 2-3, November 2000 (2000-11), Seiten 195-207, XP000989714 ISSN: 0273-2289
- KELLY J J ET AL: "Optimized target-group discrimination using a DNA microarray format to identify nitrifying species" ABSTRACTS OF THE GENERAL MEETING OF THE AMERICAN SOCIETY FOR MICROBIOLOGY, Bd. 101, 2001, Seiten 502-503, XP009036243 & 101ST GENERAL MEETING OF THE AMERICAN SOCIETY FOR MICROBIOLOGY; ORLANDO, FL, USA; MAY 20-24, 2001 ISSN: 1060-2011

## Beschreibung

Die Erfindung bezieht sich auf ein Verfahren zur Detektion von DNA-Punktmutationen (SNP-Analyse), unter Ausnutzung einer Bindung (Hybridisierung) von nachzuweisender Ziel-DNA an auf einem DNA-Chip positionsspezifisch immobilisierter Fänger-DNA.

Die DNA-Analyse mittels Hybridisierungstechnik ist ein bekanntes Verfahren (in "Gentechnische Methoden" G.Gassen und G.Schrimpf, Spektrum Akademischer Verlag Heidelberg, 1999, Kap. 11 "Blottingverfahren und Hybridisierungen", Seiten 243 bis 261). Auf einem festen Trägermaterial werden DNA-Sonden-Moleküle, sog. Fänger-Oligonukleotide immobilisiert, die aufgrund ihrer spezifischen Affinität mit der komplementären Proben-DNA, diese "einfangen" indem sie sog. Hybride, d.h. Paare von Fänger- und Zielmolekül, bilden. Dieses Bindungsereignis wird üblicherweise durch optische oder auch enzymatische Reportermoleküle angezeigt.

Anwendung für solche DNA-Analysen ist z.B. die Detektion von Infektions-Erregern, wie Tuberkulose oder HIV. Eine besondere Anforderung an die DNA-Analytik wird bei sog. "Single Nucleotide Polymorphism", kurz SNP's, gestellt. Hier ist es erforderlich, dass ein Fängermolekül, das aus ca. 20 verschiedenen Nukleotiden besteht, Zielmoleküle, die sich in nur einem einzigen Nukleotid unterscheiden, selektiv bindet bzw. nicht bindet. Da die bindungsenergetischen Unterschiede sehr klein sind, sind die Anforderungen an die Selektivität des DNA-Sensors sehr hoch.

DNA-Sensoren sind vom Stand der Technik bekannt, wozu beispielsweise auf die nichtvorveröffentlichte DE 102 59 820 A1 und die DE 102 59 821 A1 des Anmelders verwiesen wird. Die Bildung der Fänger/ziel-DNA-Hybride erfolgt unter spezifischen Randbedingungen, wobei zueinander passende Fänger/Ziel-DNA-Paare eine höhere Bindungsenergie haben als solche, die eine Fehlbasenpaarung besitzen. Aufgrund der geringen Bindungsenergieunterschiede bei SNPs lassen sich "perfect match" und "single point mismatch" oftmals nicht eindeutig unterscheiden.

Letzteres Problem wurde bisher dadurch gelöst, dass bei den finalyseverfahren des Standes der Technik ein sog. "stringenter Waschschritt" eingeführt wurde, d.h. die Ionenstärke einer Waschflüssigkeit wurde so gewählt, dass die zunächst unspezifisch gebundenen "single base mismatch" Zielmolküle von den Fängern getrennt werden, die "perfect match" Zielmoleküle jedoch an den Fängermolekülen gebunden bleiben. Ebenso sind noch aufwendigere optische Schmelzpunktanalysen möglich. Bei diesem verfahren nutzt man die intrinsische Veränderung der Lichtabsorption beim Schmelzen des DNA-Doppelstranges und es ist kein optischer Label notwenig. Sowohl beim stringenten Waschen als auch bei der optischen Schmelzpunktanalyse, bei der außerdem relativ große Mengen an DNA benötigt werden und ein Spektrophotometer für die Detektion in flüssiger Phase unerlässlich ist, können die Bedingungen meist nur auf eine einzige SNP eingestellt werden. Befinden sich mehrere SNPs auf einem Sensor-Chip, so ist die Trennung aller Fehlpaarungen nicht möglich.

Bei der optischen Detektion von Schmelzkurven ist oftmals die erforderliche Stabilität des optischen Signals (intrinsische Aktivität der DNA, bzw. Label) für kontinuierliche Messungen, bzw. Wiederholungsmessungen nicht gegeben. Das Gleiche gilt für irreversible Detektionsverfahren. Insbesondere kann es erforderlich sein, dass der Chip nach dem stringenten Waschen getrocknet werden muss, bevor er einer optischen Auslesung zugeführt werden kann

In "An Active Microelectronics Device for Multiplex DNA Ana-lysis", M. Heller, IEEE Engineering in Medicine and Biology, March/April 1996, Seiten 100 bis 104 wird weiterhin eine sog. "elektrische Stringenzbehandlung" beschrieben, bei der die Hybridisierung auf einem mit Elektroden versehenen Chip vorgenommen wird. Single-pointmismatch Hybride werden aufgrund des Poly-Anionen-Charakters der DNA durch negative Polarisierung der Elektroden getrennt. Dieses Verfahren hat sich jedoch nicht als robustes und allgemeines Verfahren etablieren können. Außerdem ist bei diesem Verfahren eine genaue Kenntnis der jeweiligen SNP-Energieunterschiede erforderlich um die individuellen elektrischen Bedingungen wie elektrisches Potential, evtl. Pulsdauer und Intensität einstellen zu können. Durch die Anwendung von energiereichen Pulsen kann die DNA beschädigt werden.

Des Weiteren ist aus der WO 02/083952 A1 ein Verfahren zur Analyse von Nukleinsäuren in einer Probe bekannt, bei dem eine SNP-Analyse durchgeführt wird und eine Hybridisierung der Proben bei vorgegebenen Temperaturen erfolgt. Die Analyse erfolgt dabei insbesondere mittels des so genannten FRET(Fluoreszenz Resonanz Energy Transfer)-Verfahrens. Die Temperaturvorgabe und Einstellung soll dabei entsprechend der US 5 965 410 A erfolgen, welche als integraler Bestandteil der WO 02/083952 A1 zitiert wird. Insbesondere wird dort ein spezifisches Programm angegeben, mit denen bei stehendendem Fluss die Temperatur auf einen bestimmten vorher errechneten Wert eingestellt wird. Des Weiteren ist aus der US 6 391 558 A ein elektrochemisches Verfahren zur Detektion von DNA bekannt, bei dem im Hybridisierungsrozess ein quantitativ auslesbares elektrisches Signal erzeugt wird.

Ausgehend von letzterem Stand der Technik ist es Aufgabe der Erfindung, ein einfaches und robustes, wie auch schonendes und reversibles Verfahren, das in einem Arbeitsgang mehrere SNPs mit unterschiedlichen, idealerweise auch unbekannten Schmelz-Temperaturen sicher detektieren kann, vorzuschlagen.

Die Aufgabe ist erfindungsgemäß durch die Abfolge der Verfahrensschritte gemäß Patentanspruch 1 gelöst. Vorteilhafte Weiterbildungen sind in den abhängigen Verfahrensansprüchen angegeben.

In spezifischer Weiterbildung macht sich das erfindungsgemäße Verfahren vorteilhafterweise die Methodik der elelctrochemischen Detektion, insbesondere des Redoxcyclings in Kombination mit einem Enzymlabel bzw. enzymatischer Verstärkung zunutze. Das dabei verwendete Enzym weist vorzugsweise eine thermische Stabilität auf. Die DNA-Fängermoleküle befinden sich auf einem festen Trägermaterial, vorzugsweise einem Silizium-Chip oder einem mit Elektroden versehenem Isolator.

Bei der erfindungsgemäßen Anordnung sind wenigstens eine Einrichtung zur Temperaturkontrolle bzw. -regelung der Flüssigkeit über den Hybridisierungspositionen des Chips sowie eine Einrichtung zur Regelung der Flüssigkeitsfließgeschwindigkeit und zugehörige Detektionsmittel vorhanden. Im Einzelnen ist dazu der Sensorchip mit einem Mikrofluidiksystem inkl. Präzisionspumpe verbunden.

Weitere Einzelheiten und Vorteile der Erfindung ergeben sich aus der nachfolgenden Figurenbeschreibung von Ausführungsbeispielen. Es zeigen
- Figur 1a, 1b und 1c: das methodische Vorgehen bei der Erfindung mit drei Beispielen für vorgegebene Temperaturprofile, einem Flüssigkeitsflussprofil sowie mit einem Sensorsignal einer einzelnen Hybridisierungsposition in Abhängigkeit von der Zeit,
- Figur 2 und 3: die aus den Sensorsignalen (Stromkurven) gemäß Figur 1c abgeleiteten Auswerte- bzw. Schmelzkurven mit den Anfangssteigungen des Stroms bzw. den auf das 40°C Signal normierten Stromanstieg als Funktion der Temperatur,
- Figur 4: eine schematisch dargestellte Anordnung zur Durchführung des Verfahrens,
- Figur 5 und Figur 6: die vergrößerte Darstellung zweier Verfahrenszustände in der Anordnung gemäß Figur 4,
- Figur 7 und Figur 8: ein Ausführungsbeispiel zur optischen Detektion beim beschriebenen SNP-Verfahrens und
- Figur 9 und Figur 10: ein Ausführungsbeispiel zur elektrochemisch enzymatischen Variante des Verfahrens.

Es soll ein Verfahren zur Detektion von DNA-Punktmutationen (SNP-Analyse), unter Ausnutzung einer Bindung, d.h. Hybridisierung, von nachzuweisender Ziel-DNA an auf einem DNA-Chip positionsspezifisch immobilisierter Fänger-DNA durchgeführt werden:

Zur vorteilhaften Realisierung der SNP-Analyse wird insbesondere folgendermaßen vorgegangen: Ein Transducer-Chip, z.B. ein Silizium-Chip oder andere, vorzugsweise planare Arrays mit mindestens einer Hybridisierungsposition und vorzugsweise elektrochemischen Transducern z.B. in Form von Mikro-Edelmetall-Elektroden wird mit mindestens einer Art von DNA-Fängersonden beladen. Die Transducer haben z.B. einen Durchmesser von 180 µm und ein zweidimensionales Rastermaß von 200x200 µm. Das Transducer-Array besitzt mehrere 10 bis ca. 100 Positionen. Zur Realisierung von SNP-Analysen werden die Sequenzen der Fängersonden so ausgewählt, dass jeweils alle vier möglichen Nukleotid-Varianten eines SNP gespottet werden. Bei genauer Kenntnis der Schmelzbedingungen von Idealpaarung (match) und Fehlpaarung (mismatch) kann auch mit einer einzigen Art von Fängersonde gearbeitet werden. Aus der Lage der Schmelzkurve kann dann auf Match oder Mismatch geschlossen werden. Um alle drei möglichen analytischen Situationen (1:nur Match, 2: nur Mismatch, 3: Match kombiniert mit Mismatch) sicher detektieren zu können, sind im einfachsten Fall zwei Messpositionen mit Match- bzw. Mismatch-Fängersonden erforderlich.

Zur praktischen Umsetzung der neuen Messmethode wird der Chip in eine Durchflusszelle eingebaut, die eine dünne Flüssigkeitsschicht über den Hybridisierungspositionen des Chips, die ein Transducer- oder Sensor-Array bilden, möglich macht. Die Hybridisierung mit der Analyt-DNA-Probe, insbesondere ein biotinyliertes PCR-Produkt, wird bei einer solchen Temperatur durchgeführt, bei der alle Match- aber auch Mismatch-Hybride entstehen können. Anschließend wird über die biotinylierten Ziel-DNA Streptavidin-Enzym-Konjugat angekoppelt.

Nunmehr wird eine Lösung mit dem für das Enzym spezifischen Substrat über den Sensor-Chip gepumpt. Die Pumpe wird für z.B. 5-10 s vorzugsweise bei konstanter Temperatur gestoppt und die Anfangssteigung des Stromanstiegs wird gemessen. Der Stromanstieg resultiert aus der Tatsache, dass das Labelenzym (z.B. eine thermostabile Esterase) das Enzymsubstrat (dem Enzym entsprechend z.B. p-Aminophenylacetat) umsetzt, das daraus entstehende Reaktionsprodukt (entsprechend p-Aminophenol) an den Transducerelektroden elektrochemisch umgesetzt wird und dadurch ein dem Reaktionsprodukt proportionaler elektrischer Strom generiert wird. Der Anstieg des elektrischen Stroms resultiert aus der Tatsache, dass das Enzym am Ort der Bindung kontinuierlich Reaktionsprodukt nachliefert und somit ein Anstieg der Produkt-Konzentration stattfindet was mit einem Anstieg des elektrischen Stroms einhergeht.

Hervorzuheben ist, dass das zu detektierende Reaktionsprodukt (z.B. p-Aminophenol) am gebundenen Labelenzym freigesetzt wird und frei diffundieren kann, bzw. mit dem Strom der Enzymsubstratflüssigkeit weggespült werden kann. Eine Detektion mittels vorzugsweise elektrochemischer Umsetzung an Elektroden oder durch optische Detektion, z.B. durch Absorptionsmessung von geeigneten optisch aktiven Reaktionsprodukten, kann nur dann erfolgen, wenn die Fließgeschwindigkeit der Enzymsubstratlösung zur Detektion deutlich reduziert wird oder vorzugsweise auf Null gesetzt wird.

Anschließend wird die Flüssigkeitspumpe wieder in Betrieb genommen (wenige µl/min) und gleichzeitig die Temperatur definiert um wenige °C, z.B. 2°C, erhöht. Durch diese Maßnahme werden zum einen die angereicherten Reaktionsprodukte von den Detektionsorten entfernt, zum anderen können die Mismatch-Ziel-Fänger-DNA-Hybride durch Temperaturerhöhung und somit Aufschmelzen gelöst und von den Hybridisierungs- bzw. Detektionspositionen wegtransportiert werden. Dadurch werden zum einen die-vorher angestiegenen elektrochemischen Signale wieder zurückgesetzt (nicht notwendigerweise auf Null, sondern lediglich deutlich reduziert) und zum anderen wird eine Re-Hybridisierung der fehlgepaarten Ziel- mit der Fänger-DNA aufgrund der Konzentrationsverminderung verhindert. Erst die zurückgesetzten Sensorsignale ermöglichen weitere Messungen. Dazu wird nach einigen Sekunden, z.B. 20 s, die Pumpe abermals für z.B. 5-10 s gestoppt und der Stromanstieg erneut aufgezeichnet.

Obige Vorgänge werden solange wiederholt, bis alle DNA-Zielmoleküle gemäß ihrer Schmelzpunkte nacheinander von den Fängersonden abgetrennt wurden. Die somit erhaltenen Schmelzkurven, d.h. die Stromanstiege aller Transducerpositionen als Funktion der Temperatur, werden ausgewertet. Dies erfolgt insbesondere rechnergesteuert nach vorgegebenem Software-Programm.

Das gesamte Verfahren ist nach ca. 10min abgeschlossen und stellt somit im Vergleich zum Stand der Technik ein besonders schnelles Verfahren dar, das aufgrund der Verwendung von enzymatischer Verstärkung eine hohe Empfindlichkeit und deshalb auch hohe Verlässlichkeit aufweist.

Ein typischer Temperaturbereich zur Aufnahme von oben beschriebenen Schmelzkurven ist ca. 40°C bis 70°C. Da Enzyme, insbesondere aus der Literatur bekannte Labelenzyme im allgemeinen nur bis ca. 40°C stabil sind, d.h. oberhalb dieser Temperatur denaturieren und somit ihre für die Messung notwendige katalytische Wirkung verlieren, werden erfindungsgemäß insbesondere thermostabile Enzyme, wie z.B. eine thermostabile Esterase eingesetzt.

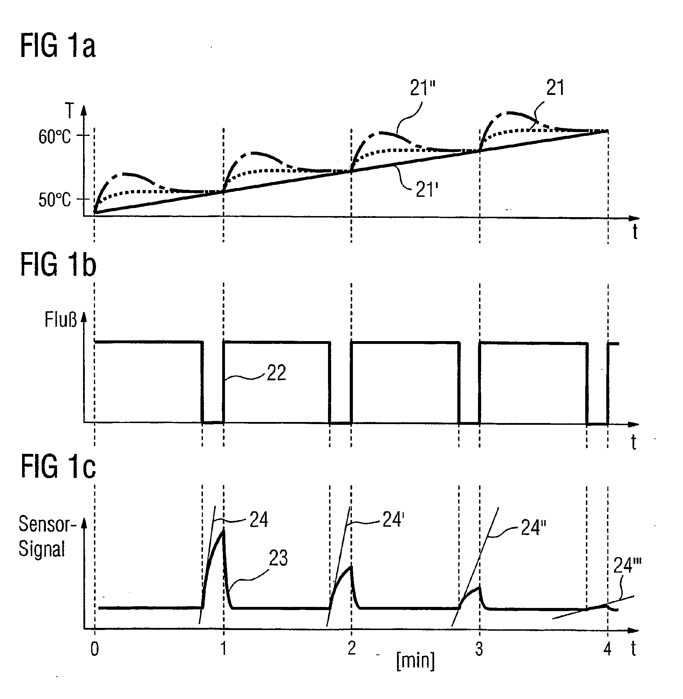

Anhand Figur 1 wird die beschriebene Messmethodik verdeutlicht. In den Teilfiguren 1a, 1b, 1c ist jeweils als Abszisse die Zeit in gleicher Skalierung aufgetragen, wobei in Figur 1a als Ordinate die Temperatur zwischen 50°C und 60°C und in Figur 1b der Fluss in nicht skalierten Einheiten und in Figur 1c das Sensorsignal (Redoxcyclingstrom) aufgetragen ist. Man erkennt, dass die Temperatur in vorgebbaren Schritten bzw. linear erhöht wird, wobei damit korreliert (synchron) der Fluss geändert wird. Vorzugsweise wird jeweils nach Einstellung einer konstanten Temperatur der Fluss geändert, insbesondere auf Null eingestellt. Sofern die Schmelztemperatur des Fänger-Ziel-DNA-Hybrides erreicht ist, wird sich die Ziel-DNA inklusive der enzymatischen Markierung unter den Gesetzen der statistischen Verteilung von der Fänger-DNA lösen und wird durch den Flüssigkeitsstrom von der jeweiligen Transducerposition entfernt und vorzugsweise in einen Abfallbehälter gespült. Die Ziel-Fänger-DNA-Hybride deren Schmelztemperatur noch nicht erreicht ist bleiben auf ihren Hybridisierungsposition erhalten. In der Waschflüssigkeit befindet sich enzymspezifisches Substrat das von den noch gebundenen Enzymlabeln zum Produkt umgesetzt wird, das seinerseits aufgrund des geänderten (insbesondere auf Null gesetzten) Flusses sich an den Hybridisierungspositionen anreichert, zu den Sensorelektroden diffundiert und elektrochemisch detektiert werden kann. In der Figur 1c ergibt sich somit ein signifikanter Anstieg des Strommesswertes, der jeweils die intakte Hybridisierung eines Fänger/Ziel-DNA-Paares kennzeichnet. Insbesondere wird die Anfangssteigung des Stromanstiegs zur Auswertung herangezogen. Wenn die Temperatur erhöht wird und der Fluss auf den Ausgangswert zurückgeführt wird, gelangt neue Flüssigkeit zu den Hybridisierungspositionen, wobei weitere Moleküle der selben Messstelle bzw. erste Moleküle von anderen Messstellen mit höherer Schmelztemperatur aufgeschmolzen und weggespült werden.

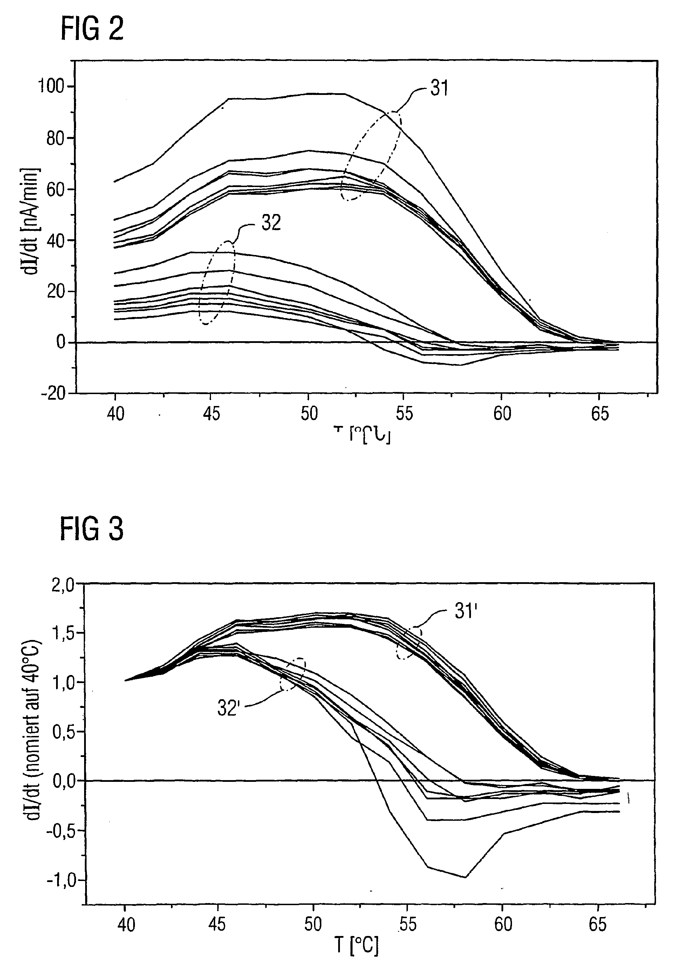

Statt der Temperaturerhöhung in Rampenschritten entsprechend Kurve 21 kann ggf. ein Temperaturanstieg auch kontinuierlich oder nach vorgegebenem Profil entsprechend den Kurven 21' bzw. 21 " erfolgen. Dabei braucht der Fluss entsprechend Kurve 22 nicht notwendigerweise gestoppt, sondern nur signifikant geändert werden. Es ergeben sich jeweils auf einander abgestimmten Profilkurven 21, 22 als Variable. Wesentlich ist die Einstellung jeweils eines stationären bzw. quasistationären Zustandes. In Figur 1c ist das Sensor-Signal, das beispielsweise als Stromwert gemessen wird, mit 23 und spezifische Steigungswerte des Sensorsignals mit 24 bezeichnet. Die Figur 2 zeigt die Schmelzkurven 31, 32 von mehreren Match-(31) sowie mehreren Single-Base-Mismatch(32)- Hybridisierungspositionen für ein Faktor-V-PCR-Produkt, die an den positionsspezifischen Transducern gemäß Figur 1c gemessen wurden, wobei der Stromanstieg dI/dt in nA/min als Funktion der Temperaturaufgetragen ist.

Die Figur 3 zeigt die aus Figur 2 normierten Messwerte Stromanstieg (T)/Stromanstieg (T=40°C)
als Kurven 31' und 32', so dass die einzelnen Kurven vergleichbar sind. Beim Faktor V im Faktor-V-PCR-Produkt handelt es sich um ein Gen, das für die Blutgerinnung von Bedeutung ist.

Im Einzelnen sind in den Figuren 2 und 3 die Anfangssteigungen der Strommesswerte (dI/dt), wie im Beispiel einer Transducer- bzw. Meßposition aus Figur 1c in Abhängigkeit vom jeweils eingestellten Temperaturwert aufgetragen. Es ergeben sich signifikante, insbesondere sigmoidale, titrationskurvenähnliche Kurvenverläufe 31, 32 für einzelne Fänger/Ziel-DNA-Paare. Wesentlich ist dabei, dass zueinander passende (match) Fänger/Ziel-DNA-Paare einen signifikant anderen Signalverlauf, bzw. eine andere Lage der Kurve entlang der Abszisse haben als nicht zueinander passende (mismatch) Fänger/Ziel-DNA-Paare. Insbesondere tritt der Schmelzvorgang und damit verbunden der Abfall der Schmelzkurve bei zueinander passenden Paaren (Match) bei höheren Temperaturen als bei nicht zueinander passenden Fänger/Ziel-DNA-Paaren (Mismatch) auf.

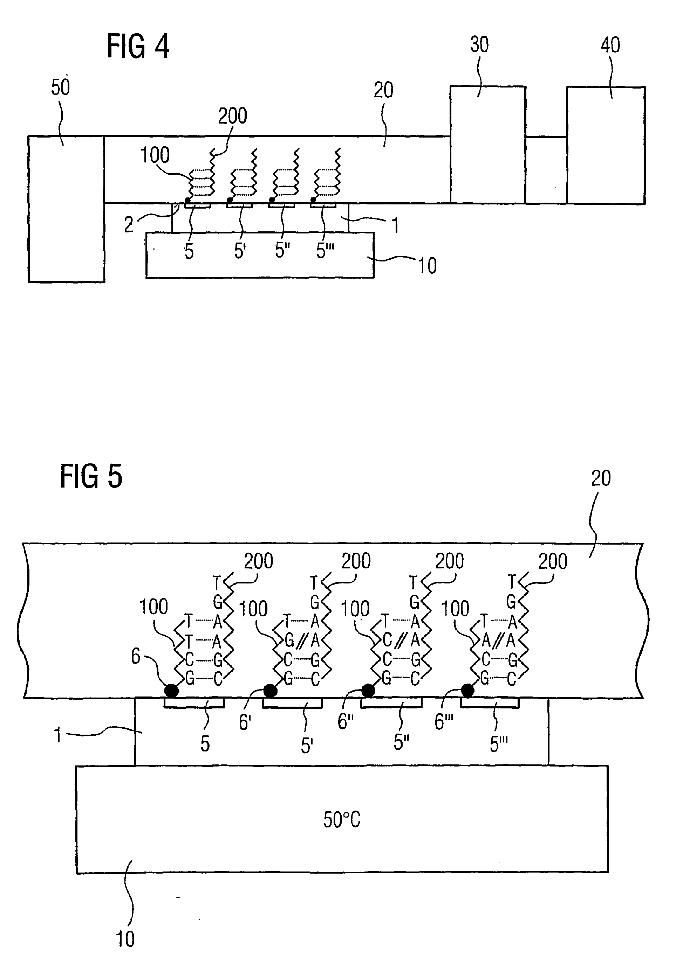

In Figur 4 ist eine allgemeine Anordnung mit einem konkret verwendeten Messaufbau, bestehend aus einem Chip mit mehreren Hybridisierungspositionen 5, 5', ..., 5ⁿ' dargestellt: In Figur 4 ist ein sogenannter DNA-Chip mit 1 bezeichnet, wie er vom Stand der Technik bekannt ist. Ein solcher DNA-Chip 1 hat auf seiner Oberfläche 2 eine Vielzahl von Messpositionen 5, 5', ..., 5ⁿ', beispielsweise in Arrayform. An jeder Messposition 5, 5', ..., 5ⁿ' sind Fänger-DNA-Moleküle immobilisiert angeordnet, beispielsweise die Fänger-DNA 100 am Immobilisierungspunkt 6. An die Fänger-DNA 100 kann sich eine Ziel-DNA 200 anlagern. Die Ziel-DNA 200 können mit einem Label versehen werden. Das Label kann ein Enzym als biokatalytische Markierung sein, wobei die Enzymmarkierung in diesem Fall vorzugsweise ein thermostabiles Enzym beinhaltet.

Die einzelnen Messstellen für die Immobilisierung der Fänger-DNA 100 sind in allen nachfolgenden Figuren mit 5, 5', ... 5ⁿ' bezeichnet. Im Chip 1, der aus Silicium oder einem anderen Halbleitermaterial gebildet ist, können vorzugsweise bereits Verstärker- und Messstrukturen, die pauschal mit 3 bezeichnet sind, eingebracht sein. Der Chip 1 kann aber auch aus einem isolierenden Material mit metallischen Elektroden ohne integrierte Signalverarbeitung bestehen.

Dem Mess-Chip 1 ist eine Einrichtung 10 zur exakten Temperatureinstellung bzw. Temperaturregelung zugeordnet. Dafür kommen beispielsweise Peltierelemente oder dergleichen in Frage. Eine Temperaturmessung erfolgt auf dem Chip und/oder gegebenenfalls auf der Einrichtung zur Temperaturregelung.

Die Oberfläche 2 als Messseite des Chips 1 ist einem Strömungskanal 20 zugewandt, durch den alle für den Analysenvorgang notwendigen Substanzen, wie Ziel-DNA 200 und ggf. Markierungs-Enzym, bzw. aus einem Reservoir 40 Waschflüssigkeit mit ggf. enzymspezifischem Substrat S mit vorgegebenem Fluss zugeführt werden. Es ist eine Flussregelung 30, die jeweils für ein vorgegebenes Zeitintervall einen exakten Fluss einhält und die einen definierten Fluss-Stopp gewährleistet, und weiterhin ein Aufnahme- bzw. Abfallbehälter 50 für nicht mehr benötigte Substanzen vorhanden.

Mit der anhand von Figur 4 beschriebenen Anordnung ist es insbesondere möglich, eine mit Enzymsubstrat S versehene Waschflüssigkeit unter präziser Temperaturkontrolle als dünne Flüssigkeitsschicht über den Chip zu führen. Dabei ist eine vorgebbare Flusskontrolle und eine genaue Messung sowie Auswertung möglich.

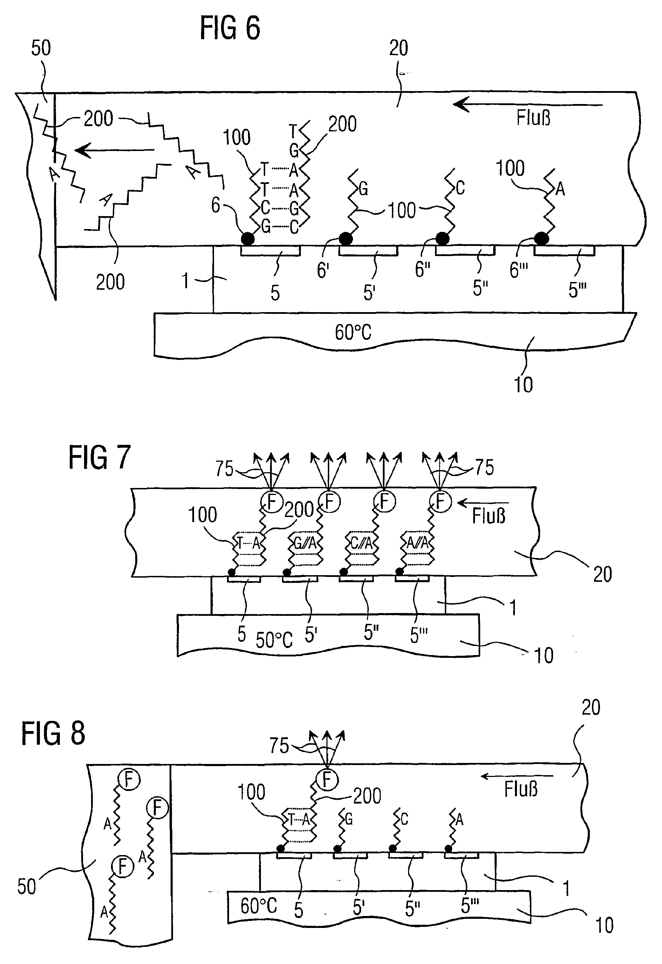

In den Figuren 5 und 6 sind zwei Verfahrens-Zustände in allgemeiner Form dargestellt, wobei vom Chip 1, der zugehörigen Thermostatierung 10 und dem Durchflusskanal 20 ausgegangen wird. In vergrößerter Darstellung sind die Fänger-DNA 100 und die Ziel-DNA 200 mit den einzelnen Polymorphismen und den zugehörigen Punktmutationen dargestellt. Insbesondere ist ersichtlich, dass beispielsweise bei einer Temperatur von 50°C alle Fänger 100 die Ziel-DNA 200 binden, wobei insbesondere die sogenannten Match-Bindungen A-T, G-C aber auch die Mismatch-Bindungen G//A, C//A und A//A vorhanden sind. Es ist offensichtlich, dass die Match-Bindungen stärker als die Mismatch-Bindungen sind.

Speziell in Figur 6 ist ein Zustand mit einer Thermostatierung von beispielsweise 60°C dargestellt, wobei bei dieser Temperatur die Mismatch-Bindungen aufgeschmolzen sind, so dass anschließend die Mismatch-Ziel-DNA weggewaschen werden. Die weggewaschenen Ziel-DNA-Moleküle können bis zum Abfallbehälter 50 gespült werden. Es ist aber auch ausreichend, die weggewaschenen Ziel-DNA-Moleküle nur eine geringe Distanz von den Mess-Position zu entfernen, sodass sie von keiner Messposition mehr detektiert werden können.

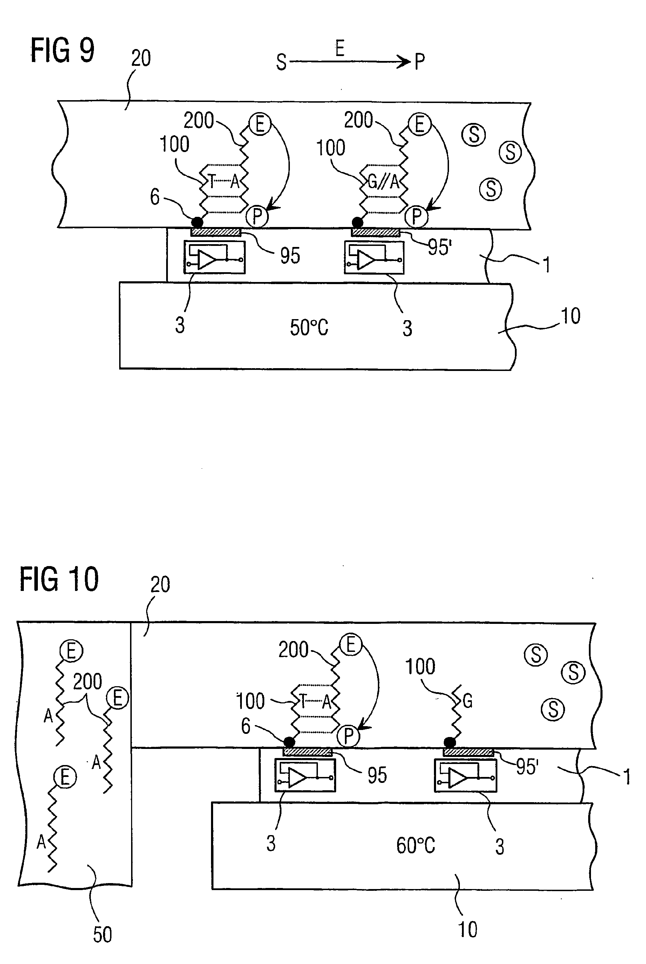

Das Aufschmelzen der Mismatch-Bindungen kann positionsspezifisch in Abhängigkeit von der Temperatur detektiert und ausgewertet werden. Dafür sind in den Figuren 7/8 einerseits und in den Figuren 9/10 andererseits zwei alternative Messmöglichkeiten dargestellt.

Die Figuren 7 und 8 gehen aus von den Figuren 4 bzw. 5/6, wobei die Ziel-DNA 200 mit einem Fluoreszenz-Label F versehen sind. Bei solcher fluoreszenzmarkierter Ziel-DNA 200 ist eine optische Auslesung möglich. Die optischen Signale 75 werden mit einem in den Figuren 7 und 8 nicht im Einzelnen dargestellten Spektrometer positionsgenau erfasst und ausgewertet.

Aus dem Vergleich der Figuren 7 und 8 ergibt sich deutlich, dass bei Überschreiten der Schmelztemperatur, beispielsweise bei der Temperatur von 60°C, die Mismatch-Bindungen geschmolzen sind und die diesbezüglichen Ziel-DNA 200 einschließlich der Fluoreszenz-Label F weggespült sind. Insofern ergibt sich bei den Mismatch-Positionen ein reduziertes Signal.

Bei der bevorzugten, alternativen enzymatisch/elektrochemischen Messung wird eine enzymatisch katalysierte Reaktion zur Bildung eines Produktes P ausgenutzt, für die folgende Gleichung gilt: 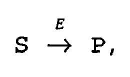 wobei S ein Enzymsubstrat, E ein Enzymlabel und P das Reaktionsprodukt bedeuten.

In der Figur 9 sind die Ziel-DNA-Moleküle 200 mit dem EnzymLabel E versehen, wobei durch die Pfeile die enzymatisch katalysierte Reaktion und die Diffusion der Reaktionsprodukte P an die elektrischen Messpositionen angedeutet ist. An den Messpositionen 5, 5', ..., 5ⁿ` des Chips 1 befinden sich elektrochemische Signalaufnehmer bzw. Transducer 95, 95', wobei in Verbindung mit den bereits erwähnten Signalverabeitungsstrukturen 3 im Silicium des Chips 1 unmittelbar ein solches elektrisches Signal wie z.B. ein Strom erfasst werden kann, welches ein Maß für die Konzentration des Reaktionsproduktes P darstellt.

Entsprechend den Figuren 7 und 8 sind in Figur 9 und Figur 10 wiederum die beiden Zustände bei einer Temperatur von 50°C und einer Temperatur von 60°C gezeigt. Es ergeben sich somit im ersten Zustand durch Umsetzung von S in P an allen Messpositionen vergleichsweise große elektrische Signale und im zweiten Zustand reduzierte bzw. keine Signale and den Mismatch-Positionen, jedoch ein vergleichsweise großes elektrochemisches Signal an der Match-Position. Die weggewaschenen Ziel-DNA-Moleküle, sowie weggewaschenes Reaktionsprodukt P können wiederum bis zum Abfallbehälter 50 gespült werden. Es kann aber auch ausreichend sein, die weggewaschenen Ziel-DNA-Moleküle und Reaktionsprodukte nur um eine geringe Distanz von den Mess-Positionen zu entfernen, so dass sie von keiner der vorhandenen Messpositionen mehr detektiert werden können.

Ein besonderer Vorteil des enzymatisch/elektrochemischen Verfahrens besteht in der Tatsache, dass es im Gegensatz zu z.B. optischen Verfahren weitgehend unabhängig von einem Hintergrundsignal ist, da der Stromanstieg dI/dt zur Auswertung herangezogen wird und nicht das absolute Stromsignal selbst. Dadurch ist es nicht nötig, dass die enymmarkierte Ziel-DNA, sowie durch sie generiertes Reaktionsprodukt P bis in den Abfallbehälter zu spülen. Es muss lediglich die messpositionsspezifische Aufkonzentration des Reaktionsproduktes P durch kurzzeitiges Spülen abgebaut werden. Dadurch ist es auch ausreichend, bei den folgenden Spülvorgängen die Waschflüssigkeit lediglich hin- und herzupumpen und es wird somit eine Einsparung von Waschflüssigkeit ermöglicht, was insbesondere bei integrierten und miniaturisierten Ausführungsformen von Vorteil ist.

Die Stromsignale entsprechen den Peaks in Figur 1c, wobei in den Figuren 2 und 3 deren Anfangssteigungen dI/dt entsprechend den Geraden 24, 24',24", ... aus Figur 1c ausgewertet werden.

Neben den beiden Beispielen mit einer Detektion über optische oder enzymatische Label ist auch eine labelfreie Detektion der gebundenen Ziel-DNA 200 möglich. Bei der optischen labelfreien Detektion werden die intrinsischen Veränderungen der UV-Absorption beim Aufschmelzen der DNA-Doppelstränge erfasst. Bei der elektrischen labelfreien Detektion wird dagegen der Prozess einer intrinsischen Guanin-Oxidation ausgenutzt. Eine weitere labelfreie Detektion kann mittels elektrochemischer Impedanzmethoden erfolgen. Weiterhin ist auch eine labelfreie Detektion durch Messungen der Massenänderung, d.h. gravimetrisch, z.B. über akustische Methoden wie Oberflächenwellen-Sensoren (sog. SAW's) möglich.

Bei den Labelverfahren ist auch der Einsatz von magnetischen Labeln in Kombination mit Magnetfeld-Sensoren möglich.

Bei den beschriebenen Methoden ist wesentlich, dass die Messung und zugehörige Auswertung automatisierbar ist. So können gelt werden. In Abhängigkeit davon erfolgt das jeweilige Flussprofil, bei dem zunächst bei Einstellung einer vorgegebenen Temperatur einerseits die aufgeschmolzenen Mismatch-Bindungen weggespült werden und andererseits die Detektion der positionsspezifischen gebundenen Ziel-DNA bei "nicht bewegter" Waschflüssigkeit erfolgt.

## Patentansprüche

1. Verfahren zur Detektion von DNA-Punktmutationen (SNP-Analyse) unter Ausnutzung einer Bindung (Hybridisierung) von nachzuweisender Ziel-DNA an auf einem DNA-Chip positionsspezifisch immobilisierter Fänger-DNA, wobei ein definierter zeitlicher Ablauf erfolgt, mit folgenden Verfahrensschritten:
- Es erfolgt bei vorgegebenem Temperaturprofil abwechselnd eine erste Phase (Waschphase) und eine zweite Phase (Messphase),
- wobei in der Waschphase
a) eine Waschflüssigkeit mit geregelter Fliessgeschwindigkeit über den DNA-Chip geleitet wird,
b) die Temperatur an den Hybridisierungspositionen als Funktion der Zeit definiert verändert wird, wobei eine stetige Temperaturerhöhung als Funktion der Zeit in Rampen mit darauffolgenden Haltezeiten der Temperatur vorgenommen wird,
c) die Fänger/Ziel-DNA-Hybride temperaturabhängig aufgeschmolzen und durch die strömende Waschflüssigkeit Ziel-DNA von den Hybridisierungspositionen entfernt werden, und
- wobei in der Messphase bei "nicht bewegter" Waschflüssigkeit eine Detektion der positionsspezifisch gebundenen Ziel-DNA erfolgt, wozu
d) der Fluss der Waschflüssigkeit während der Temperaturhaltezeiten gestoppt wird,
e) bei der aktuellen Temperatur noch gebundene Ziel-DNA positionsspezifisch detektiert wird,
f) die Signale nach vorgegebenem Programm ausgewertet werden.

2. Verfahren nach Anspruch 1, **dadurch gekennzeichnet, dass** die Verfahrensschritte a) bis e) mehrfach wiederholt werden.

3. Verfahren nach Anspruch 1 oder Anspruch 2, **dadurch gekennzeichnet, dass** die Detektion der gebundenen Ziel-DNA labelfrei (markierungsfrei) erfolgt.

4. Verfahren nach Anspruch 3, **dadurch gekennzeichnet, dass** die labelfreie Detektion optisch erfolgt.

5. Verfahren nach Anspruch 4, **dadurch gekennzeichnet, dass** die optische Detektion die intrinsische Veränderung der UV-Absorption beim Aufschmelzen von DNA-Doppelsträngen nutzt.

6. Verfahren nach Anspruch 3, **dadurch gekennzeichnet, dass** die labelfreie Detektion elektrisch erfolgt.

7. Verfahren nach Anspruch 6, **dadurch gekennzeichnet, dass** die labelfreie Detektion mittels der intrinsischen Guanin-Oxidation erfolgt.

8. Verfahren nach Anspruch 7, **dadurch gekennzeichnet, dass** die labelfreie Detektion mittels elektrochemischer Impedanzmethoden erfolgt.

9. Verfahren nach Anspruch 7, **dadurch gekennzeichnet, dass** die labelfreie Detektion gravimetrisch erfolgt.

10. Verfahren nach Anspruch 1 oder 5, **dadurch gekennzeichnet, dass** die Detektion der gebundenen Ziel-DNA unter Ausnutzung eines Labels (Markierung) erfolgt.

11. Verfahren nach Anspruch 10, **dadurch gekennzeichnet, dass** die Detektion mittels eines optischen Labels erfolgt.

12. Verfahren nach Anspruch 10, **dadurch gekennzeichnet, dass** die Detektion mittels eines magnetischen Labels erfolgt.

13. Verfahren nach Anspruch 12, **dadurch gekennzeichnet, dass** die Detektion mittels eines enzymatischen Labels erfolgt.

14. Verfahren nach Anspruch 13, **dadurch gekennzeichnet, dass** die Waschflüssigkeit Enzymsubstrat enthält.

15. Verfahren nach Anspruch 14, **dadurch gekennzeichnet, dass** ein im verwendeten Temperaturbereich stabiles aktives Enzym verwendet wird.

16. Verfahren nach den Ansprüchen 12 bis 15, **dadurch gekennzeichnet, dass** das enzymatische Label eine Reaktion katalysiert die optisch detektierbar ist.

17. Verfahren nach den Ansprüchen 12 bis 15, **dadurch gekennzeichnet, dass** das enzymatische Label eine Reaktion katalysiert die elektrochemisch detektierbar ist.

18. Verfahren nach Anspruch 17, **dadurch gekennzeichnet, dass** es sich bei dem elektrochemischen Detektionsverfahren um eine mittels Redoxcycling verstärkte Strommessung handelt.

19. Verfahren nach den Ansprüchen 12 bis 15, **dadurch gekennzeichnet, dass** die Veränderung der Konzentration des Substrates oder Produktes detektiert und ausgewertet wird.

20. Verfahren nach einem der vorhergehenden Ansprüche, **dadurch gekennzeichnet, dass** die Auswertung und Darstellung des Sensorsignals (Detektionssignal) als Funktion der Temperatur erfolgt.

21. Verfahren nach Anspruch 20, **dadurch gekennzeichnet, dass** bei der Auswertung eine Normierung der Schmelz-Kurve auf eine bestimmte Temperatur erfolgt.

22. Verfahren nach einem der vorhergehenden Ansprüche, **dadurch gekennzeichnet, dass** die Auswertung rechnergesteuert wird.

23. Verfahren nach Anspruch 22, **dadurch gekennzeichnet, dass** die Auswertung softwaremäßig durchgeführt wird.

24. Verfahren nach einem der vorangehenden Ansprüche, wobei eine Anordnung verwendet wird, welche folgende Merkamle aufweist:
- wenigstens einen DNA-Chip (1) mit Fänger/Ziel-DNA-Hybriden,
- eine Waschflüssigkeit und zugehörige Messmittel,
- eine Einrichtung (20) zum lateralen Anströmen der Chip-Oberfläche (2) mit Waschflüssigkeit,
- eine Einrichtung (30) zur Flusskontrolle der Waschflüssigkeit,
- eine Einrichtung (10) zur Temperatursteuerung oder - regelung, und
- Mittel (1 - 5, 75, 95) zur Detektion der Ziel-DNA.

25. Verfahren nach Anspruch 24, wobei der DNA-Chip (1) Transducer (5, 5', ...; 95) als Teile eines Arrays zur Immobilisierung von DNA-Fängersonden (100) auf der Chip-Oberfläche (2) bildet.

26. Verfahren nach Anspruch 24, wobei die Waschflüssigkeit ein Enzymsubstrat (S) enthält.

27. Verfahren nach Anspruch 26, wobei Enzymlabel (E) als Markierung für die Ziel-DNA (100) vorhanden sind.

28. Verfahren nach Anspruch 27, wobei die Enzymlabel (E) thermostabil sind.

29. Verfahren nach Anspruch 24, wobei die Einrichtung (10) zur Temperatursteuerung bzw. -regelung ein zeitlich vorgebbares Temperaturprofil einstellt.

30. Verfahren nach Anspruch 24, wobei die Einrichtung (30) zum Anströmen des DNA-Chips (1) mit Waschflüssigkeit mit vorgegebenem Flussprofil in Abhängigkeit von der eingestellten Temperatur regelbar ist.

31. Verfahren nach Anspruch 24, wobei die Detektionsmittel (1 - 5, 95) zur elektrochemischen Detektion des enzymatischen Umsatzes ausgebildet sind.

## Claims

1. Method for detecting DNA point mutations (SNP analysis) utilizing a binding (hybridization) of target DNA to be detected to capture DNA which is immobilized position-specifically on a DNA chip, where a defined time sequence takes place, with the following method steps:
- a first phase (washing phase) and a second phase (measurement phase) take place alternately with a predetermined temperature profile,
- where in the washing phase
a) a washing liquid is passed at a controlled flow rate over the DNA chip,
b) the temperature at the hybridization positions is changed in a defined manner as a function of time, with a continuous increase in temperature as a function of time being carried out in ramps with subsequent temperature holding times,
c) the capture/target DNA hybrids are melted temperature-dependently and target DNA are removed from the hybridization positions by the flowing washing liquid, and
- where in the measurement phase with the washing liquid "not in motion", the position-specifically bound target DNA is detected, for which purpose
d) the flow of washing liquid is stopped during the temperature holding times,
e) target DNA still bound at the current temperature is detected position-specifically,
f) the signals are analyzed according to a predetermined program.

2. Method according to Claim 1, **characterized in that** method steps a) to e) are repeated more than once.

3. Method according to Claim 1 or Claim 2, **characterized in that** the detection of the bound target DNA takes place without label (label-free).

4. Method according to Claim 3, **characterized in that** the label-free detection takes place optically.

5. Method according to Claim 4, **characterized in that** the optical detection makes use of the intrinsic change in the UV absorption on melting of DNA double strands.

6. Method according to Claim 3, **characterized in that** the label-free detection takes place electrically.

7. Method according to Claim 6, **characterized in that** the label-free detection takes place by means of the intrinsic guanine oxidation.

8. Method according to Claim 7, **characterized in that** the label-free detection takes place by means of electrochemical impedance methods.

9. Method according to Claim 7, **characterized in that** the label-free detection takes place gravimetrically.

10. Method according to Claim 1 or 5, **characterized in that** the detection of the bound target DNA takes place with use of a label (labeling).

11. Method according to Claim 10, **characterized in that** the detection takes place by means of an optical label.

12. Method according to Claim 10, **characterized in that** the detection takes place by means of a magnetic label.

13. Method according to Claim 12, **characterized in that** the detection takes place by means of an enzymatic label.

14. Method according to Claim 13, **characterized in that** the washing liquid comprises enzyme substrate.

15. Method according to Claim 14, **characterized in that** an active enzyme which is stable in the temperature range used is used.

16. Method according to Claims 12 to 15, **characterized in that** the enzymatic label catalyzes a reaction which can be detected optically.

17. Method according to Claims 12 to 15, **characterized in that** the enzymatic label catalyzes a reaction which can be detected electrochemically.

18. Method according to Claim 17, **characterized in that** the electrochemical detection method is a redox cycling-amplified current measurement.

19. Method according to Claims 12 to 15, **characterized in that** the change in the concentration of the substrate or product is detected and analyzed.

20. Method according to any of the preceding claims, **characterized in that** the analysis and representation of the sensor signal (detection signal) takes place as a function of temperature.

21. Method according to Claim 20, **characterized in that** the melting curve is standardized to a defined temperature in the evaluation.

22. Method according to any of the preceding claims, **characterized in that** the analysis is computer-controlled.

23. Method according to Claim 22, **characterized in that** the analysis is carried out by software.

24. Method according to any of the preceding claims, in which an arrangement having the following characteristics is used:
- at least one DNA chip (1) with capture/target DNA hybrids, a washing liquid and associated measurement means,
- one device (20) for supplying the chip surface (2) laterally with washing liquid,
- one device (30) for monitoring the flow of washing liquid,
- one device (10) for controlling the temperature, and
- means (1-5, 75, 95) for detecting the target DNA.

25. Method according to Claim 24, in which the DNA chip (1) forms transducers (5, 5', ...; 95) as parts of an array for immobilizing DNA capture probes (100) on the chip surface (2).

26. Method according to Claim 24, in which the washing liquid comprises an enzyme substrate (S).

27. Method according to Claim 26, in which enzyme labels (E) are present as labeling for the target DNA (100).

28. Method according to Claim 27, in which the enzyme labels (E) are thermostable.

29. Method according to Claim 24, in which the device (10) for controlling the temperature sets up a temporally predeterminable temperature profile.

30. Method according to Claim 24, in which the device (30) for supplying the DNA chip (1) with washing liquid with a predetermined flow profile can be controlled as a function of the temperature which is set.

31. Method according to Claim 24, in which the detection means (1-5, 95) are configured for the electrochemical detection of the enzymatic conversion.

## Revendications

1. Procédé de détection de mutations ponctuelles d'ADN ( analyse de PNS ) en exploitant une liaison ( hybridation ) d'un ADN cible à déceler avec de l'ADN de captation immobilisé en des positions spécifiques sur une puce à ADN suivant un déroulement défini dans le temps, comportant des étapes de procédé suivantes consistant à :
- réaliser alternativement une première phase ( phase de lavage ) et une deuxième phase ( phase de mesure ) à un profil de température prescrit,
- dans la phase de lavage,
a ) amener un liquide de lavage sur la puce à ADN à une vitesse d'écoulement régulée,
b ) modifier la température aux positions d'hybridation de façon définie en fonction du temps, une élévation continue de la température en fonction du temps étant effectuée sous la forme de rampes qui comportent des durées successives de maintien de la température,
c ) fondre les hybrides ADN de captation/ADN cible en fonction de la température et éliminer l'ADN cible des positions d'hybridation par le liquide de lavage en écoulement, et
- dans la phase de mesure, effectuer avec un liquide de lavage « non déplacé » une détection de l'ADN cible lié à des positions spécifiques, ce pour quoi
d ) on arrête le flux de liquide de lavage pendant les durées de maintien de la température,
e ) à la température instantanée, on détecte à des positions spécifiques de l'ADN cible encore lié,
f ) on exploite les signaux conformément à un programme prescrit.

2. Procédé selon la revendication 1, **caractérisé en ce que** les étapes de procédé a ) à e ) sont répétées plusieurs fois.

3. Procédé selon la revendication 1 ou la revendication 2, **caractérisé en ce que** la détection de l'ADN cible lié se fait sans marqueur.

4. Procédé selon la revendication 3, **caractérisé en ce que** la détection sans marqueur se fait par des moyens optiques.

5. Procédé selon la revendication 4, **caractérisé en ce que** la détection optique utilise la variation intrinsèque de l'absorption des UV lors de la fusion des doubles brins d'ADN.

6. Procédé selon la revendication 3, **caractérisé en ce que** la détection sans marqueur se fait par des moyens électriques.

7. Procédé selon la revendication 6, **caractérisé en ce que** la détection sans marqueur se fait par oxydation intrinsèque de la guanine.

8. Procédé selon la revendication 7, **caractérisé en ce que** la détection sans marqueur se fait au moyen de procédés électrochimiques à impédance.

9. Procédé selon la revendication 7, **caractérisé en ce que** la détection sans marqueur se fait par gravimétrie.

10. Procédé selon la revendication 1 ou 5, **caractérisé en ce que** la détection de l'ADN cible lié se fait en utilisant un marqueur.

11. Procédé selon la revendication 10, **caractérisé en ce que** la détection se fait au moyen d'un marqueur optique.

12. Procédé selon la revendication 10, **caractérisé en ce que** la détection se fait au moyen d'un marqueur magnétique.

13. Procédé selon la revendication 12, **caractérisé en ce que** la détection se fait au moyen d'un marqueur enzymatique.

14. Procédé selon la revendication 13, **caractérisé en ce que** le liquide de lavage contient un substrat enzymatique.

15. Procédé selon la revendication 14, **caractérisé en ce que** l'on utilise un enzyme actif qui est stable dans la gamme de températures utilisée.

16. Procédé selon les revendications 12 à 15, **caractérisé en ce que** le marqueur enzymatique catalyse une réaction qui est détectable par des moyens optiques.

17. Procédé selon les revendications 12 à 15, **caractérisé en ce que** le marqueur enzymatique catalyse une réaction qui est détectable par des moyens électrochimiques.

18. Procédé selon la revendication 17, **caractérisé en ce que** le procédé de détection électrochimique est une mesure de courant amplifiée par cycle Redox.

19. Procédé selon les revendications 12 à 15, **caractérisé en ce que** l'on détecte et l'on exploite la variation de la concentration du substrat ou du produit.

20. Procédé selon l'une des revendications précédentes, **caractérisé en ce que** l'exploitation et la représentation du signal de capteur ( signal de détection ) se font en fonction de la température.

21. Procédé selon la revendication 20, **caractérisé en ce que**, lors de l'exploitation, l'on norme la courbe de fusion sur une température déterminée.

22. Procédé selon l'une des revendications précédentes, **caractérisé en ce que** l'exploitation est commandée par ordinateur.

23. Procédé selon la revendication 22, **caractérisé en ce que** l'exploitation est effectuée par logiciel.

24. Procédé selon l'une des revendications précédentes, dans lequel on utilise un système qui présente les éléments caractéristiques suivants :
- au moins une puce à ADN ( 1 ) qui comporte des hybrides ADN de captation/ADN cible,
- un liquide de lavage et des moyens de mesure associés,
- un dispositif ( 20 ) destiné à amener latéralement un liquide de lavage sur la surface ( 2 ) de la puce,
- un dispositif ( 30 ) destiné à contrôler le flux du liquide de lavage,
- un dispositif ( 10 ) destiné à commander ou réguler la température, et
- des moyens ( 1 à 5, 75, 95 ) destinés à détecter l'ADN cible.

25. Procédé selon la revendication 24, dans lequel la puce à ADN ( 1 ) est constituée de transducteurs ( 5, 5' ,...; 95 ) qui font partie d'un réseau destiné à immobiliser des sondes de captation d'ADN ( 100 ) sur la surface ( 2 ) de la puce.

26. Procédé selon la revendication 24, dans lequel le liquide de lavage contient un substrat enzymatique ( S ).

27. Procédé selon la revendication 26, dans lequel des marqueurs enzymatiques sont utilisés pour marquer l'ADN cible ( 100 ).

28. Procédé selon la revendication 27, dans lequel les marqueurs enzymatiques ( E ) sont thermostables.

29. Procédé selon la revendication 24, dans lequel le dispositif ( 10 ) de commande ou de régulation de la température règle un profil de température prescrit dans le temps.

30. Procédé selon la revendication 24, dans lequel le dispositif ( 30 ) destiné à amener un liquide de lavage sur la puce à ADN ( 1 ) avec un profil d'écoulement prescrit peut être réglé en fonction de la température réglée.

31. Procédé selon la revendication 24, dans lequel les moyens de détection ( 1 à 5, 95 ) sont conformés de façon à détecter la conversion enzymatique par des moyens électrochimiques.
